(19) 
Europäisches Patentamt
European Patent Office
Office européen des brevets

(11) **EP 3 854 298 A1**

(12) **EUROPEAN PATENT APPLICATION**

(43) Date of publication:
**28.07.2021 Bulletin 2021/30**

(51) Int Cl.:
**A61B 5/00** *(2006.01)*          **A61B 5/0205** *(2006.01)*
**A61B 5/11** *(2006.01)*          **G08B 21/02** *(2006.01)*

(21) Application number: **20152864.3**

(22) Date of filing: **21.01.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **HILBIG, Rainer**
  **5656 AE Eindhoven (NL)**

• **BULUT, Murtaza**
  **5656 AE Eindhoven (NL)**
• **BOSMA, Robert Willem**
  **5656 AE Eindhoven (NL)**
• **CHEN, Shuang**
  **5656 AE Eindhoven (NL)**
• **SHI, Jun**
  **5656 AE Eindhoven (NL)**

(74) Representative: **Philips Intellectual Property & Standards**
**High Tech Campus 5**
**5656 AE Eindhoven (NL)**

(54) **A SYSTEM AND METHOD FOR ALERTING A CAREGIVER BASED ON THE STATE OF A PERSON IN NEED**

(57)    The invention provides a system for alerting a second subject (e.g. a parent) based on a wellbeing state of a first subject (e.g. a child) when the first subject is sleeping in the same room as the second subject. An arrangement of sensors is used for sensing data from the first subject and a processor is configured to determine the presence of the first subject from the sensing data. Based on the first subject being present, the processor is further configured to determine the state of the first subject based on the sensing data from the arrangement of sensors and then determine whether to alert the second subject based on the state of the first subject. An alarm is provided for alerting the second subject.

FIG.2

EP 3 854 298 A1

**Description**

FIELD OF THE INVENTION

The invention relates to a system and method for improving sleep quality when a person in need and a caregiver(s) are sleeping in the same room by alerting the caregiver(s) based on the state of the person in need.

BACKGROUND OF THE INVENTION

Quite often toddlers join their parent's bed in the sleeping room of the parents for a common sleep. This normally results with sleep interruption of at least one of the parents and also with a high risk that the child will also be disturbed by the now restless sleeping parents. This will result for the whole family in not having an energized start of the next day.
Unobtrusive monitoring of a child sleeping alone and providing the parents processed information is well known. Even the option to give some feedback to the child is realized. One example for this is the Philips baby phone Philips Avent SCD 630 that uses audio and video information to check the status of the child and allows for feedback by changing light levels, playing different kind of music as well as speaking with the child via the device.
Other inventions are known which optimize the interaction of children with their parents based on the sleep phases of the parents. When a newborn starts to wake up at night, only the parent who is in lightest sleep, or the one who is needed (e.g. breast feeding mom at feeding moments), will be alarmed gently (e.g. vibration) by analyzing and comparing both the parent's and the baby's sleep state (awake, light sleep or deep sleep.). Optionally further elements are taken into account to decide who to wake up, such as each parent's sleep journey so far, activities next day, level of fatigue, etc.
However, these systems do not take into account a child co-sleeping with the parents. Co-sleeping may be sleeping in the same bed, sleeping in a different bed but in the same room or just generally sleeping in the same room at the start of the night or as needed throughout the night. Co-sleeping may have many benefits to both the parents and the child, as it reduces separation anxiety for the child and also reduces the time spent by the parents nursing the child back to sleep.
It may seem at first that there is no need for unobtrusive child monitoring when the child is in the same room as the parents. However, mental and physical wellbeing of the parents and the child could be much improved if neither of the parents had to wake up each time the child needs attention.
Sleeping in the same room may also occur between a person in need and a caregiver. The caregiver may be helping a person with an impairment, such as due to old age, disability or mental disorder. Sleeping in the same room may be beneficial as it drastically improves the time taken for the caregiver to come to aid if needed. However, sleeping in the same room in this case may also reduce the sleep quality of both the person in need and the caregiver, which may affect the mood and efficacy of the caregiver negatively.
Therefore, there is a need for a system which can improve the quality of sleep of caregiver(s) and people in need when they are sleeping in the same room.

SUMMARY OF THE INVENTION

The invention is defined by the claims.
According to examples in accordance with an aspect of the invention, there is provided a system for alerting a second subject based on a wellbeing state of a first subject when the first subject is sleeping in the same room as the second subject, comprising:

an arrangement of sensors for sensing data from the first subject;
a processor configured to:

determine the presence of the first subject from the sensing data;
based on the first subject being present, determine the state of the first subject based on the sensing data from the arrangement of sensors; and
determine whether to alert the second subject based on the state of the first subject; and
an alarm for alerting the second subject.

The system continuously senses, through an arrangement of sensors, the parameters relating to a first subject, which is usually a child, when it is sleeping in the same room as a second subject, usually a parent. A processor then uses the data from the arrangement of sensors to determine a wellbeing state of the child. For example the state of the child could be whether it is awake or asleep, whether the child has enough space and/or whether the child is crying. If the processor determines the child is in an unwanted state, it alerts the parent through an alarm that the child is in an unwanted state.

The "wellbeing state" (in this document generally abbreviated to just "state") of a subject is thus one or more measures of wellbeing of the subject. These measures may relate to their sleep state, the space they have, whether they are calm or agitated, and measures of physiological parameters (such as temperature, respiration rate, movement characteristics, heart rate, etc.).. Thus, the wellbeing state in the case of a child is indicative of whether the child needs some care or assistance (such as feeding, needing more space, needing medical attention etc.).

The system can further be configured such that the sensors can sense when the child is in the room. This is useful when the child moves to sleep in the room with the parent after the parent has gone to sleep, so the parent might not be aware that the child is in the bed. The processor is also configured to only process the data from the sensors when the child is detected.

The arrangement of sensors may be further configured to sense data for the second subject and the processor may be further configured to determine a wellbeing state of the second subject. The processor may be configured to determine whether to alert the second subject further based on the state of the second subject.

The state of the parent can also be determined from the data of the sensors by the processor. The processor can then decide whether to alert the parent of the state of the child based on the state of the parent. For example if the child does not have enough space, but it is not urgent for the parent to move, the processor might decide to wait until either the parent is more awake or until it is more urgent for the child to have more space, e.g. when the child starts crying.

Thus, the wellbeing state may not simply be a binary indication (such as awake/asleep, or cramped/not cramped) but also a measure of degree.

The invention also provides a system for alerting a third subject when the third subject is sleeping in the same room as the first and second subject. The system further comprises a second alarm for alerting the third subject. The arrangement of sensors is further configured to sense data for the third subject and the processor is further configured to determine a wellbeing state of the third subject, wherein the processor is configured to determine whether to alert one or more of the second subject and the third subject further based on the state of the third subject.

The system can also accommodate for two parents sleeping the same bed. In this case each parent also has a separate alarm. Similarly to the system with one parent, the state of the third subject, e.g. the second parent, is determined and the processor decides whether to alert the parents based on the state of the child and the states of each of the parents. For example, processor can choose to alert either or both of the parents based on which parent is closer to the child, which parent may be needed (e.g. breastfeeding) or which of the parents is in a better state to take care of the child.

A user interface may be used for receiving user preferences from one or more of the second subject and the third subject, wherein the determining whether to alert one or more of the second subject and the third subject is further based on the user preferences.

The system can be set up such that the parents can input user preferences before sleeping. The user preferences can include which parent to wake up more often, which parent to wake up at which time window or which days to wake up which parent.

A memory unit can be used for storing historic data from one or more of the arrangement of sensors and the processor, wherein the determining whether to alert one or more of the second subject and the third subject is further based on the historic data.

A memory unit can be used to store historic data from either or both of the sensors and the processor. The decisions made by the processor could be used in conjunction with the sensed data immediately after the decision. This data can be used in the future by the processor to determine which decision is best to make dependent on the situation. For example, if the second parent is likely to wake up after 5am when the first parent gets an alert, then it might be beneficial to mostly alert the second parent after 5am. The data from the sensors therefore helps the system to learn how each of the subjects responds to the alerts in certain situations.

The arrangement of sensors may be further adapted to determine the sleep stage of one or more of the first subject, the second subject and the third subject, wherein the determining whether to alert one or more of the second subject and the third subject is further based on the sleep stage of one or more of the first subject, second subject and the third subject.

The sleep stage of the child and the sleep stage of the parents can also be used to determine which parent to alert. In one example, when the child is sensed to be present and both of the parents are in deep sleep, the processor may decide to wait until one of the parents is in light sleep, but if the child then enters light sleep, then the processor might further decide to alert the parent furthest away from the child in order not to disturb the child. In another example, if the child is awake but not crying, only the parent in the lightest sleep stage is alerted.

The arrangement of sensors may further comprise monitoring sensors for monitoring the first subject, the monitoring sensors comprising one or more of:

a temperature sensor;
a movement sensor;
a respiration sensor; and
a heart rate sensor,
wherein the determining whether to alert one or more of the second subject and the third subject is further based on the monitoring sensor signals.

For example, if the temperature of the child is determined to be too low by the processor, one of the parents can be alerted to possibly cover the child up, close a window or increase the temperature of an air conditioning unit.

The processor may further control a temperature management system, such as an air conditioning unit, based on the temperature of the first subject.

The system may also comprise of a plurality of types of alerts, wherein the processor is further configured to select the type of alert to be actuated by an alarm based on the state of the first subject.

There may be a plurality of alerts available, such as sound alerts, vibration alerts and lighting alerts. There can also be many different messaging alerts dependent on the state, sleep stage and temperature of child, as well as of the parent to be alerted. The processor can decide which alert is best suited for different situations. For example, a message stating "Child is cold" on a smart watch or mobile phone, in conjunction with gradual vibrations may be used to alert a parent that the child is cold.

The invention also provides a method for alerting a second subject based on the state of a first subject, comprising:

determining the presence of a first subject;
determining a wellbeing state of the first subject;
determining whether to alert a second subject based on the state of the first subject; and
in response to determining whether to alert the second subject, actuating an alarm for alerting the second subject.

The method may further comprise:

determining a wellbeing state of the second subject;
determining a wellbeing state of a third subject;
determining whether to alert one or more of the second subject and the third subject based on the state of one or more of the first subject, the second subject and the third subject; and
in response to determining whether to alert one or more of the second subject and the third subject, actuating one or more of a first alarm for alerting the second subject and a second alarm for alerting the third subject.

The method may also further comprise:

receiving user preferences;
reading historic data from a memory unit;
determining the temperature of the first subject; and
determining whether to alert one or more of the second subject and the third subject further based on one or more of the user preferences, the historic data and the temperature of the first subject.

The method for determining whether to alert one or more of the second subject and the third subject may further comprise:

determining the sleep stage of any one of the first subject, the second subject and the third subject; and
determining whether to alert one or more of the second subject and the third subject further based on the sleep stage of one or more of the first subject, second subject and the third subject.

The method may further comprise selecting an alert to be actuated by an alarm based on the state of the first subject.

The invention also provides a computer program comprising code means for implementing any of the methods mentioned above when said program is run on a processing system.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a system which monitors a first subject as well as a second and third subject sleeping in the same bed;
Figure 2 shows a first schematic representation of the inputs a processor uses to decide which subject to wake up;
Figure 3 shows a second schematic representation of the inputs a processor uses to decide which family member to wake up;
Figure 4 shows how the sleep cycle data may be used to determine when to alert a subject and which subject to alert; and
Figure 5 shows a method used to alert a second subject of the state of the first subject.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for alerting a second subject (e.g. a parent) based on the state of wellbeing of a first subject (e.g. a child) when the first subject is sleeping in the same room as the second subject. An arrangement of sensors is used for sensing data from the first subject and a processor is configured to determine the presence of the first subject from the sensing data. Based on the first subject being present, the processor is further configured to determine the state of the first subject based on the sensing data from the arrangement of sensors and then determine whether to alert the second subject based on the state of the first subject. An alarm is provided for alerting the second subject.

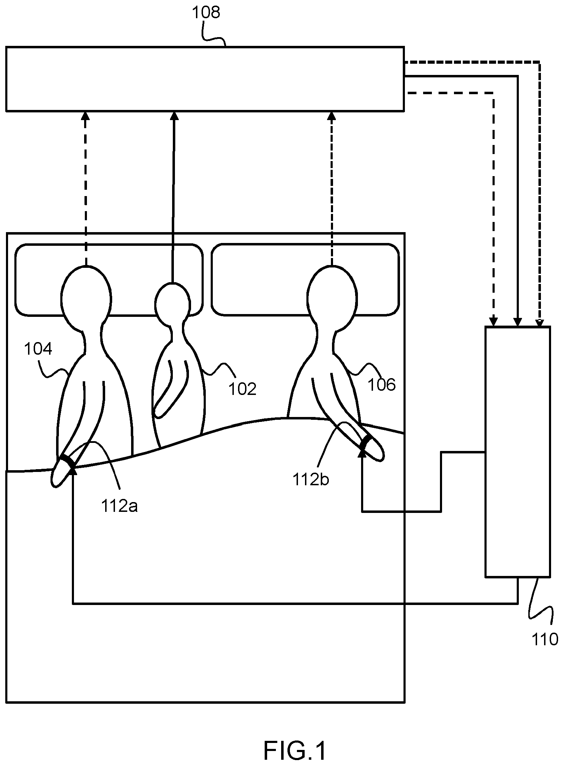

Figure 1 shows a system which monitors a first subject 102 (e.g. a child) as well as a second subject 104 and third subject 106 (e.g. parents) sleeping in the same bed. The child 102 may have began the night sleeping in the same bed as the parents 104, 106 or the child 102 may have begun the night sleeping in a different room and moved to sleep in the parents 104 106 room during the night.

An arrangement of sensors 108 can continuously monitor the room to sense the presence of the child 102. When the sensors 108 detects the presence of the child 102 in the room, the state of the child 102 is calculated from the data sensed by the sensors 108 by a processor 110. The state of the child 102 may include: the child 102 is fine, the child 102 is cold, the child 102 does not have enough space etc. When the child 102 is first detected, a message may sent to the parents 104, 106 to make them aware of the presence of the child 102.

If the state of the child 102 is negative (e.g. the child is cold), the processor may send a signal to one (or both) of the parents to alert them of the state of the child. For example if the child does not have enough space because one of the parents 104 is too close, the processor 110 may send a message to the parent 104 to alert them that they need to give the child more space. This "negative" state of the child may thus be any of the possible measures of wellbeing which indicate that the child needs assistance. Thus, various different measures of wellbeing may be combined to arrive at an individual measure which can be used to decide whether or not an alarm is needed.

The messages may be sent though alarms 112a and 112b. These alarms may be, for example, wearable devices and/or smart phones.

The system may also work similarly for a child sleeping in the same room as the parents, but in a different bed, or for a person in need sleeping in the same room as a caregiver.

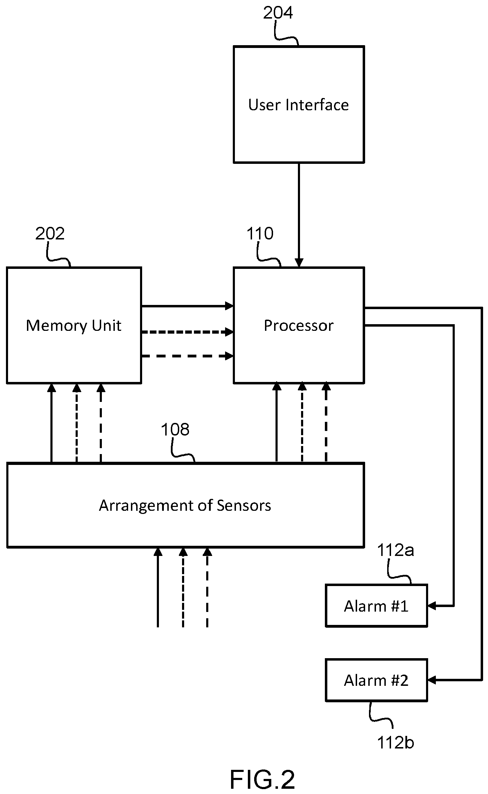

Figure 2 shows a first schematic representation of the inputs a processor uses to decide which subject to wake up. The processor 110 uses the data for each of the subjects (e.g. child 102, first parent 104 and second parent 106), the user preferences and historic sleep data to decide whether to alert either or both parents 104, 106 and when to alert them.

The user preferences can be input by the parents 104 106 in a user interface 204. The user interface 204 may be an application on a smart phone, a standalone device which is configured to receive user preferences or an existing device (such as a smart watch) which can be configured to receive user preferences. The user preferences may include whether one parent does not want to be woken up on a specific night, which times are best to wake which parent up etc.

The historic sleep data is stored in a memory unit 202 and is the data from the arrangement of sensors 108 from previous nights. The historic sleep data may include room temperatures, a hypnogram for each night for each of the subjects and movement from each of the subjects. From the historic sleep data it can be inferred by the processor 110 which parent 104 106 is best to alert for certain situations. For example, the first parent 104 may be more likely to wake up to an alert between 1am and 3am.

When the processor 110 has chosen which parent to alert, it sends a message to one of the alarms 112a 112b. The message may be one or more of, a text message, an audio message or a vibrational alert.

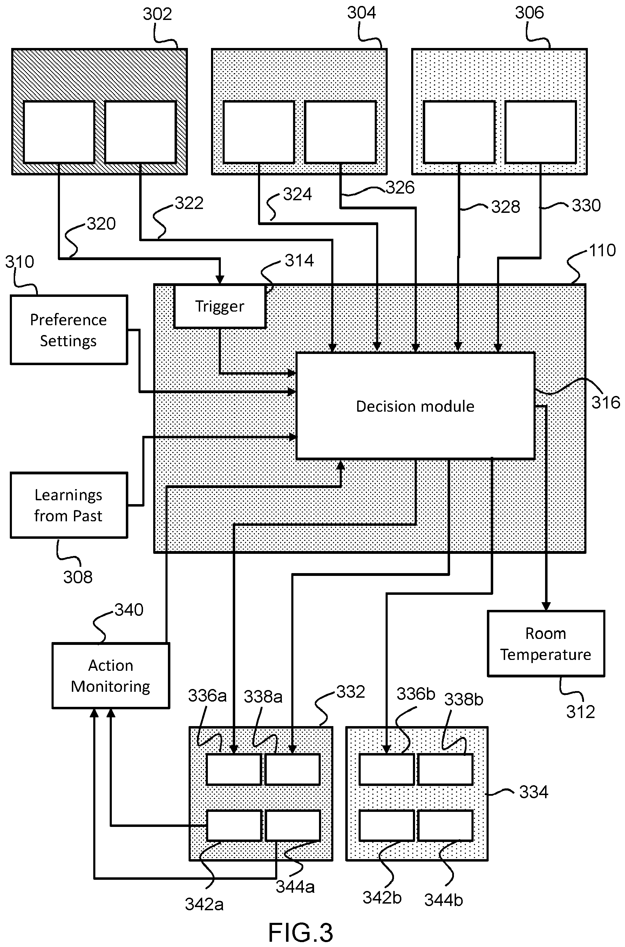

Figure 3 shows a second schematic representation of the inputs a processor uses to decide which family member to wake up. The processor 110 (e.g. a decision module) uses sensed data of the whole family (e.g. child data 302, father data 304, mother data 306) in bed as well as learnings from the past 308 and user preferences 310 to derive and control suitable actions. The processor 110 indicates the presence of the child 102 in the bed to at least one parent 104, 106 as well as alerting the parents if the child 102 is in need at a determined moment in time taking the boundary condition "good sleep for all members of the family" into account. Accompanying actions like adapting room temperature 312 (e.g. air conditioning) settings or playing relaxing music are applied to further optimize sleep quality and well-being of the family. The age of the child 102 in view may be between just born and about 10 years; decisions derived take actual age into account.

The care for the child 102 (i.e. well covered, sufficient place, awake and needs to be calmed) at suited time (with respect to sleep phases of the parents and needs of the child) taking the boundary condition "good sleep for all members of the family" into account is described below in ten steps:

1. Presence of child 320 (as starting trigger 314 when presence of child in parents bed is monitored), position and behavior of child, movements and sleep status of every family member, child is speaking or child starts crying are monitored by suited sensors.

2. Sensed data (e.g. activity data 322 of the child, movement data 324,328 of the parents and sleep data 326, 330) are sent at all times to a decision module 316.

3. If the presence of the child (signal 320) activates the trigger 314, sensed data is analyzed by the decision module 316.

4. The decision module 316 may indicate the position of the child, whether the child is well covered or not, whether the child is awake, whether the child is restless, whether the child starts crying etc. It also monitors sleep status, position and movements of the parents.

5. The decision module 316 decides on which parent to be addressed and the time delay for a message about the child status and/or needed child care action.
The decision for on which parent to awake is based on sleep phase of each parent (through a sleep tracker 344a for the father and a sleep tracker 344b for the mother), preference settings 310 and learnings from the past 308 on the addressability of the corresponding parent.
The decision for the time delay for a message is based on the urgency of the care case and (prolonging) options like adapting room temperature settings 312 that do not require parent support.

6. At selected moments in time, the decision module 316 checks that message content further holds true. If yes, it addresses one of the devices (alarms 112) to which the selected parent is assigned. The device selection depends on message content. In general, messages provided by this device should not disturb the child and the other parent. For example, the devices could be an alarm watch 336a for the father and a mobile phone 338a for the father and an alarm watch 336a for the mother and a mobile phone 338b for the mother.

7. If needed (when actions are required) the decision module 316 checks, with help of the sensed data, that an action is taken through action monitoring 340.

8. If no action is monitored (e.g. through a movement sensor 342a for the father or a movement sensor 342b for the mother), step 5 is started again. Another message delivering device may be assigned or the other parent may be alerted. The order and frequency depend on urgency of care case and learnings from the past.

9. As long as child care takes place (monitored action of one parent), no further message delivery takes place.

10. Steps 4 - 9 are performed as long as the child is present in the bed 320 unless the processor 110 is deactivated. Deactivation can be manually switching off but also can be externally triggered.

Both parents are thus sensed with movement sensors 342 and sleep sensors 344 and the child is monitored via presence detection 320 and an activity monitor 322. The data for the sensors may be inputs for the decision module 316. The decision module 316 outputs (messages) may be given to alarms 112 (e.g. output modules 336b, 338b, 336a and 338a). Output modules are devices that translate the message of the decision module 316 into a characteristic signal for the addressed parent.

An important aspect is how the status of the child is communicated to (one of) the parents and how the message is followed up by the parent(s). Below four examples are given in quotations to explain the kind of communication in more detail. They are ordered corresponding to the urgency of message delivery and required action.

Just to inform parents

If the decision module 316 finds "child in your bed, but all ok", a message may only be delivered to each of the parents in the case of either of the parents becoming awake (delayed delivery).

Action needed, but sufficient time left

In case of the decision module 316 finding "child not covered", a message may be provided slightly delayed in case neither of the parents is awake. The maximum delay is determined by whether an action can be taken without the need of a parent (e.g. closing a window or turning heating on to increase room temperature) and how fast the child 102 gets into a uncomfortable situation (using ambient room temperature as measure or body temperature as direct measure). The delay is given by a set-value which is adapted by learning from the past. When the maximum delay time is reached, one parent will be alerted (preferably the one in the lighter sleep phase).

Action (e.g. parent covers child again) is checked (e.g. via camera monitoring). If no reaction is made within a certain time window (e.g. a few minutes), a next message will be sent, such as to the other parent.

Action needed, but only (short) time delay until reaction

In case of the decision module 316 finding "child in unrest", the delay of the message is determined from prior learnings 308 as well as from the user preferences 310. For example, the system can learn how long it takes before the child 102 starts to cry when it wakes up, as the parents may have the subjective feeling that the child 102 needs care if it is fluttering for more than 20 seconds. At least one parent is woken up after the time delay, preferably the parent in the lighter sleep phase.

The action by the parent (parent awakes and takes care of child 102) is checked. If no reaction by the parent is sensed within a certain time window (e.g. less than one minute), another message will be sent (perhaps to the other parent).

Action directly needed

In case of the decision module 316 finding "not sufficient space" due to parent's movement and position of the child, a direct message may be sent to that parent that is responsible for the child having less space. In a first step, this message may be sent only to the father in the typical way of message communication. If no reaction is monitored within a few seconds, a more annoying message is sent (e.g. higher vibration intensity). If there is no direct reaction to the second message, the other parent may be alerted.

The importance (and sequence of message delivery) can be correlated to the remaining space for the child 102 and the learning from how the father normally behaves with respect to "reducing space for child".

It may also be possible to change the shape of the mattress with help of actuators. The mattress may change shape to attempt to move the parent to give the child more space. This may also be done by vibrating the mattress.

If only one parent is messaged, the message has to be delivered in a manner that the other parent is not disturbed, if possible.

Examples of messages include: suitable sound from a smart phone or other device with speaker(s) (the mother may not be disturbed by Mozart music at low volumes, but the father may wake up) and vibration alarms (such as from a smart watch, a smart phone or an under-mattress vibration pad) where the kind of vibration is an indication of what is the content of the message.

Parents may not use identical output devices for the message communication.

For important messages, the reaction of the parents which has been messaged is monitored.

At least one parent might not be aware that the child 102 has entered the bed during the night. To adapt the sleep behavior for this parent it is important that the parent becomes aware of the presence of the child 102.

The decision of which parent to wake up depends on the monitored sleep phase and known (learned) sensitivity to personal alarms and user preferences 310 (like do not disturb mother this night).

Windows may be closed (and air conditioner started) by the system to adapt the temperature of the child 102 or to reduce the child's allergies.

Presence and movement detection of persons can be done by using one or more of a camera system, ultrasound and radar devices, motion detectors, force sensors (on, in, under the matrasses, for example), microphones, wearable devices and smart phones.

Changes in the vital signs of the co-habitants may also be used to infer information about the different occupants. For example, the average (body or skin) temperature may be larger when multiple people are in the bed, in comparison to when sleeping alone. The differences will be larger when three people are present than when two people are present. In addition to the average temperature (or other parameter), also the general rate of change will be different. For example, the temperature may increase faster when more people are present.

Similarly, it can be expected that the respiration patterns of one person will be influenced by the respiration patterns of the other people in the bed. The same may also be observed expected for heart rate patterns.

Another way of monitoring presence is by observing changes in the vital signs of one subject, as a result of other subject moving. For example, if a parent moves, the smooth and regular breathing pattern of a child will be disturbed.

Thus, by analyzing the interaction between multiple signals (especially around the change events) presence can be detected.

Different mechanisms may be used to track the sleep cycle (sleep phases of persons):

a headband that detects brainwaves to track sleep cycles and an algorithm which is used to optimize sleep cycle tracking;
a motion camera to track an increase, a decrease and the duration of motion during the night;
a microphone used to track sound levels which can track movement in bed;
a heart rate monitor (because heart rate differs between sleep cycles);
a respiration sensor for monitoring breathing patterns;
an algorithm to analyze the relation between breathing and heart patterns;
a gas detector for CO2 sensing;
user log tags which can be used to track sleep disturbers and force sensors which can be used to track breathing.

Relationships between different parameters may be analyzed, such as the relationship between respiration and movement patterns, between heart rate and movement patterns, and between respiration, heart rate, and movement patterns.

Presence and movement detection of devices (e.g. the bed covers) can be done by using one or more of a camera system, ultrasound and radar devices, force sensors (e.g. on, in, under the bed covers) and temperature sensors (e.g. on, in, under the bed covers).

Communication between sensors 108, output modules (e.g. alarms 112) and decision module 316 may be done by using one or more of: a direct cable connection of the sensors and output devices with the decision module 316, wireless communication via Bluetooth and communication via a (wireless) network.

Output modules for message delivery may be one or more of: a smart phone 338, an alarm clock, audio devices, illumination and signaling systems, wearable devices 336 with vibration function (or alternative alarming functions), stationary devices with vibration or movement function (e.g. on, in, under the mattress).

Depending on the urgency of message delivery, the intensity and the content of the message may be different. Three cases are considered as examples:

audio message (using a device with loudspeaker);
vibrational alarm;
using two output devices together (e.g. vibrational alarm followed by audio message).

For the case of an audio message: four cases are considered as examples:

just to inform parents "Child is in your bed, all ok", provided at low sound level (close to addressed parent);
action needed but sufficient time left, the message "Child lost cover" may be provided at low sound level (close to addressed parent);
action needed but only (short) time delay until reaction, the message "Child woke up and is restless" may be provided at increased sound level (close to addressed parent); and
action directly needed, the message "Child needs space" may be provided at high sound level (close to addressed parent).

For the case of a vibrational alarm (e.g. on worn wristband), four cases are considered as examples:

just to inform parents, a one second vibration may be provided at low amplitude;
action needed but sufficient time left, a one second vibration followed by a one second pause and further followed by a one second vibration may be provided at low amplitude;
action needed but only (short) time delay until reaction, a one second vibration of low intensity followed by a one second pause further followed by a one second vibration at a medium intensity may be provided;
action directly needed, a continuous vibration with increasing amplitude may be provided.

Using two output devices together (e.g. vibrational alarm followed by spoken message) is also possible. For example, a vibrational alarm may be used as described beforehand together with the spoken message. However, the audio message may be delivered at a low volume in all cases to achieve less disturbance to the other parent. Another example is to use an audio message when a parent is awake and to use a vibrational alarm to wake up a parent.

The system can also learn which signal is best suited for alarming either parent and how each parent reacts to each alarm. The system can also learn which signal impacts each partner the least.

Below are derived a set of relations that may be used by the decision module 316 to decide on a) which parent to be addressed and b) the time delay $t_{del}$ for a message about the child status and/or needed child care action. These relations depend on the urgency of message delivery.

The following abbreviations are used during this description:

| | |
|---|---|
| Specific parent: | father or mother |
| Time of event at child: | $t_0$ |
| Time delay for a message: | $t_{del}$ |
| Time a parent awakes: | $t_2$ |
| Time of missed action of one parent: | $t_{missed}$ |
| Time delay for a specific case Cx: | $t(Cx)$ |
| Time delay before repeat attempt: | $dt(Cx)$ |
| Preference settings to become wake | $A_{parent}$ |
| Learnings for addressability: | $B_{parent}$ |
| Sleep status data of parents: | $sleep(parent)$ |
| Allowance to disturb or not: | $C_{parent}$ |

In case of only one parent present, the following descriptions will only address this parent, and the algorithm may be adapted correspondingly.

Just to inform parents

$t_{del}(father) = "\infty"$; $t_{del}(mother) = "\infty"$;
event "father awakes" triggers $t_{del}(father) = 0$ s, message is directly provided to father; $\rightarrow$ delay time set to $t_{del}(father) = "\infty"$
event "mother awakes" triggers $t_{del}(mother) = 0$ s, message is directly provided to mother $\rightarrow$ delay time set to $t_{del}(mother) = "\infty"$.

Content of message is checked at the moment of delivery, if situation "just to inform parents" is no longer true, this message will not be delivered.

Action needed but sufficient time t(C2) left

$t_{del}(father) = t(C2)$; $t_{del}(mother) = t(C2)$;

a) One parent (e.g. father) awakes at $t_2 < t_0 + t(C2) \rightarrow$ event "father awakes" triggers $t_{del}(father) = 0$ s, message is directly provided to father $\rightarrow$ action of father monitored $\rightarrow$ delay time set to $t_{del}(father) = "\infty"$ and $t_{del}(mother) = "\infty"$;
No action of father within 30 s $\rightarrow t_{del}(father) = t(C2) - (t_{missed} - t_0)$; $t_{del}(mother) = t(C2) - (t_{missed} - t_0)$, where tmissed $= t_2 + 30s$, and repeat a).
b) Neither father nor mother wakes up at $t < to + t(C2) \rightarrow$ the most addressable parent is woken up. The decision of addressability $D_{add}$ uses the status data Sadd(parent) that are derived from the sleep status of father and mother together with set data $A_{parent}$ and learnings from the past ($B_{parent}$). $S_{add}(parent)$ is given by the relation:

$$S_{add}(parent) = A_{parent} * B_{parent} * sleep(parent) + C_{parent}$$

$$D_{add} = S_{add}(father) - S_{add}(mother)$$

$D_{add} < 0 \rightarrow$ mother addressed, $D_{add} > 0 \rightarrow$ father addressed, $D_{add} = 0 \rightarrow$ random function used.

Where the values for $A_{parent}$ $B_{parent}$ and $C_{parent}$ are derived from the user preferences and what the system has learnt over previous nights

Action needed but only (short) time delay (t(C3)) until reaction

a) One parent awakes at $t < t_0 + t(C3) \rightarrow$ event "father awakes" triggers $t_{del}(father) = 0$ s, message is directly provided to father $\rightarrow$ action of father monitored $\rightarrow$ delay time set to $t_{del}(father) = "\infty"$ and $t_{del}(mother) = "\infty"$;
b) Neither father nor mother awakes at $t < t_0 + t(C3) \rightarrow$ At $t = t_0 + t(C3)$ the most addressable parent is woken up.

Attempt to awake the selected parent (father) by a suitable message: event "father to wake" triggers $t_{del}(father) = 0$ s, message is directly provided to father $\rightarrow$ action of father monitored $\rightarrow$ delay time set to $t_{del}(father) = "\infty"$ and $t_{del}(mother) = "\infty"$;
No action of father after finalization of message delivery $\rightarrow$ tdei(father) = dt(C3); $t_{del}(mother)$ = dt(C3) and repeat b).

Action directly needed

a) Direct action needed, but not an interaction with a specific parent $\rightarrow$ At t = to (i.e. directly) the most addressable parent is woken up. The decision of addressability $D_{add}$ uses the status data Sadd(parent).
In case of no reaction after dt(C4), the other parent is also alerted. Alerting the parents is stopped when care action is monitored $\rightarrow$ delay time set to $t_{del}(father) = "\infty"$ and $t_{del}(mother) = "\infty"$;
b) Direct action needed from a specific parent (father) $\rightarrow$ father is directly selected ($D_{add} > 0$) $\rightarrow$ action of father monitored $\rightarrow$ delay time set to $t_{del}(father) = "\infty"$ and $t_{del}(mother) = "\infty"$

In case of no reaction of father after dt(C4), the mother is also alerted.
Addressing the parents is stopped, when care action is monitored$\rightarrow$ delay time set to $t_{del}(father) = "\infty"$ and $t_{del}(mother) = "\infty"$;
Below are derived the relationships that are used to determine the following:

| | |
|---|---|
| Time delay for a specific case Cx: | t(Cx) |
| Time delay before repeat attempt: | dt(Cx) |
| Preference settings to become wake: | $A_{parent}$ |
| Learnings for addressability: | $B_{parent}$ |
| Allowance to disturb or not: | $C_{parent}$ |
| Sleep status data of parents: | sleep(parent) |

The given times in the relations are exemplary values for the order of useful times.

$$t(C1) = "\infty"$$

$$t(C2) = age * set * learn(C2) * 15 \text{ min}$$

$$t(C3) = age * set * learn(C3) * 2min$$

$$t(C4) = 0 \text{ s}$$

The parameters age (based on age of the child in months), set (0 hour-settings, may be default values or input by parents from the user preferences) and learn (adapted factor due to the system learning during use) can be defined by:

$$\text{Age} = (\text{Age of the child in months} / 5) = 0.1$$

For children younger than 10 months: Age = (Age of child in month / 5) + 0.1
For older children: Age = 2.1
Set = between 0.3 and 3
learn(Cx, 0 hours) = 1;
if in prior event at $t_o$ child was further on not restless at $t_o + t(Cx) \rightarrow$ learn(Cx, new) = 1.1 * learn(Cx, old);
if in prior event at to child was restless before $t_o + t(Cx) \rightarrow$ learn(Cx, new) = 0.9 * learn(Cx, old).

Typical time delays before repeat attempt may be:

$dt$ (C1) = "∞"
$dt$ (C2) ~ 1 minute
$dt$ (C3) ~ 10 seconds
$dt$ (C4) ~ 5 seconds

Preference settings to become wake: $A_{parent}$

The 0-hour set value may be 0.6. This preference value $A_{parent}$ can be varied individually for each parent between $A_{parent}$ = 0.3 to $A_{parent}$ = 1. This can be done manually but can also be supported by known schedule (tomorrow crowded working day → decrease $A_{parent}$ by 0.2 points (if possible)).

The setting $A_{parent}$ can also be used to clearly indicate a preferred parent to address (e.g. breast-feeding mother).

Learnings for addressability: $B_{parent}$

The 0-hour set value of $B_{parent}$ may be 0.8. This value is adapted by the decision module using the learnings of the past. Input is the addressability of father or mother in case of being asked for care due to an event of the child.

In case of not reacting after the first given message → $B_{parent}$ (new) = $B_{parent}$ (old) - 0.1, if $B_{parent}$ (old) > 0.6.

In case of reacting after the first given message → $B_{parent}$ (new) = $B_{parent}$ (old) + 0.1, if $B_{parent}$ (old) < 1.

Allowance to disturb or not: $C_{parent}$

The 0-hour set value of $C_{parent}$ may be 0. This value is set manually in the user preferences for both parents. The value for a parent is set equal to 1 if a parent does not want to be woken up. The value $C_{parent}$ is automatically set back to 0 after the night.

Sleep status data of parents: sleep(parent)

The value of sleep(parent) is varied over night corresponding to the individual sleep phases of the parents (which are sensed by suitable sleep phase sensors and communicated to the decision module). Below are exemplary factors listed for the different sleep phases.

Awake   → sleep(parent) = 1
Light sleep   → sleep(parent) = 0.7
Deeper sleep   → sleep(parent) = 0.4
Very deep sleep   → sleep(parent) = 0.2

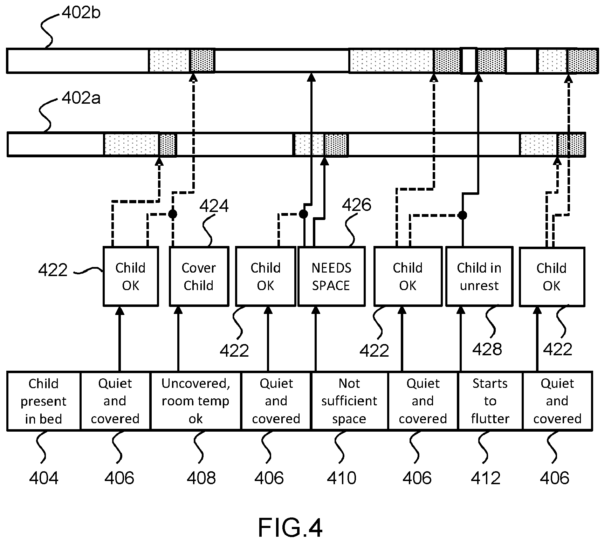

Figure 4 shows how the sleep cycle data may be used to determine when to alert a subject (e.g. a parent) and which subject to alert. The focus of the system is on the state of the child and the needed care, and not on the sleep phases 402 of the parents. The sleep phases 402 of the parents are only taken into account to better illustrate delays in information delivery and to illustrate which parent is better suited to take care of the child 102 at certain times. For example, the sleep phases of the father 402a and the sleep phases of the mother 402b could be determined simultaneously.

In this example, the presence and activity of child 102 (and cover presence) as well as activity of parents is monitored via a camera system. The sleep status of the parents 402 is monitored using activity watches. The vibration function of the watches is used as output devices.

A description of the time evolution is given below following the child status that is monitored with help of sensors as function of time:

Child (age: 0.5 years) present in parents double bed 404 → system 2 monitors via camera that child is present → analyzation of all sensed data is started.

Child quiet and is well covered 406 → message "Child OK" 422 for parents ("1 s vibration", provided at low amplitude) is prepared, sleep phase of parents is monitored → message is given to father's watch as he awakes, message to mother pending.

Child quiet, but camera monitoring shows that child is not well covered 408 (but temperature is ok → case 1) → pending message to mother is not delivered; instead message "Cover Child" 424 ("1 s vibration, 1 s pause, 1 s vibration", provided at low amplitude) is delivered to the watch of the mother as mother wakes up. Mother awakes at $t_2 < t_0 + t(C2)$. In this case $t(C2) = age * set * learn(C2) * 15 min = 0.5 * 2 * 2 * 15 min = 30 min$.

Child sleeps and is well covered 406 → message "Child OK" 422 for parents (" 1 s vibration", provided at low amplitude) is prepared, sleep phase of parents is monitored → message to mother and father pending.

Child does not have sufficient space 410 → movements of father monitored via camera → pending message 422 to parents is not delivered; instead instant message "NEEDS SPACE" 426 to father ("continuous vibration with increasing amplitude"), no reaction of father indicated within $dt(C4) = 3s$ → instant message "NEEDS SPACE" 426 ("continuous vibration with increasing amplitude") to both parents → action of father monitored → message to parents stopped.

Child quiet and is well covered 406 → message "Child OK" 422 for parents (" 1 s vibration", provided at low amplitude) is prepared, sleep phase of parents is monitored → message is given to mother's watch as she awakes, message to father pending.

Child starts to flutter (as monitored via the camera) 412 → pending message 422 to father is not delivered; instead message "Child in unrest" 428" (1 s vibration (low amplitude), 1 s pause, 2 s vibration (medium amplitude)) is delivered to the watch of that parent (mother) that is selected via addressability $D_{add}$. The message is delivered at $t = to + t(C3)$ and action of mother is monitored.

$$t(C3) = age * set * learn(C3) * 2min = 0.5 * 2 * 1 * 2 min = 1 min$$

$$S_{add}(father) = A_{father} * B_{father} * sleep(father) + C_{father} = 0.4*0.7*0.2 + 0 = 0.056$$

$$S_{add}(mother) = A_{mother} * B_{mother} * sleep(mother) + C_{mother} = 0.6*0.8*0.4 + 0 = 0.192$$

$$D_{add} = S_{add}(father) - S_{add}(mother) = 0.056 - 0.192 = \textbf{-0.136} \rightarrow mother$$

Child quiet and is well covered 406 → message "Child OK" 422 for parents (" 1 s vibration", provided at low amplitude) is prepared, sleep phase of parents is monitored → message is given to father's watch as he awakes, and to mother's watch as she awakes later.

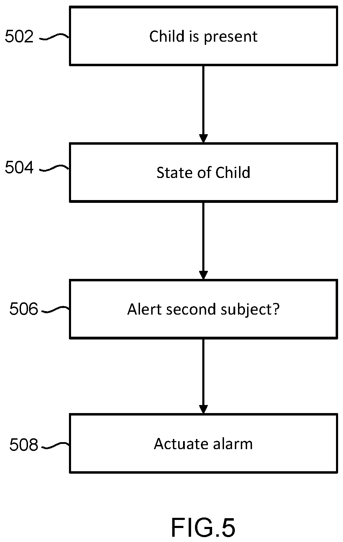

Figure 5 shows a method used to alert a second subject of the state of the first subject. First, it is determined whether the child is present in the room in step 502. Once the presence of the child is detected, the state of the child is determined in step 504. Depending on the state of the child which has been determined, it is determined whether to alert a second subject, step 506. If it is determined that the second subject must be alerted, an alarm is actuated in step 508 to alert the second subject.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A system for alerting a second subject (104) based on a wellbeing state of a first subject (102) when the first subject (102) is sleeping in the same room as the second subject (104), comprising:

   an arrangement (108) of sensors for sensing data from the first subject;
   a processor (110) configured to:

   determine the presence of the first subject (102) from the sensing data;
   based on the first subject (102) being present, determine the state of the first subject (102) based on the sensing data from the arrangement of sensors; and
   determine whether to alert the second subject (104) based on the state of the first subject; and
   an alarm (112a) for alerting the second subject.

2. A system as claimed in claim 1, wherein the arrangement (108) of sensors is further configured to sense data for the second subject (104) and the processor is further configured to determine a wellbeing state of the second subject (104), wherein the processor is configured to determine whether to alert the second subject further based on the state of the second subject.

3. A system as claimed in claim 2 further for alerting a third subject when the third subject is sleeping in the same room as the first and second subject, comprising a second alarm for alerting a third subject, wherein the arrangement of sensors is further configured to sense data for the third subject and the processor is further configured to determine a wellbeing state of the third subject, wherein the processor is configured to determine whether to alert one or more of the second subject and the third subject further based on the state of the third subject.

4. A system as claimed in claim 3, further comprising a user interface for receiving user preferences from one or more of the second subject and the third subject, wherein the determining whether to alert one or more of the second subject and the third subject is further based on the user preferences.

5. A system as claimed in any of claims 3 or 4, further comprising a memory unit for storing historic data from one or more of the arrangement of sensors and the processor, wherein the determining whether to alert one or more of the second subject and the third subject is further based on the historic data.

6. A system as claimed in any of claims 3 to 5, wherein the arrangement of sensors is further adapted to determine the sleep stage of one or more of the first subject, the second subject and the third subject, wherein the determining whether to alert one or more of the second subject and the third subject is further based on the sleep stage of one or more of the first subject, second subject and the third subject.

7. A system as claimed in any of claims 3 to 6, wherein the arrangement of sensors further comprise monitoring sensors for monitoring the first subject, the monitoring sensors comprising one or more of:

   a temperature sensor;
   a movement sensor;
   a respiration sensor; and
   a heart rate sensor,
   wherein the determining whether to alert one or more of the second subject and the third subject is further based on the monitoring sensor signals.

8. A system as claimed in claim 7, wherein the processor further controls a temperature management system, such as an air conditioning unit, based on the temperature of the first subject.

9. A system as claimed in any of the preceding claims, further comprising a plurality of types of alerts, wherein the processor is further configured to select the type of alert to be actuated by an alarm based on the state of the first subject.

10. A method for alerting a second subject based on the state of a first subject, comprising:

(502) determining the presence of a first subject;
(504) determining a wellbeing state of the first subject;
(506) determining whether to alert a second subject based on the state of the first subject; and
(508) in response to determining whether to alert the second subject, actuating an alarm for alerting the second subject.

11. A method as claimed in claim 10, further comprising:

determining a wellbeing state of the second subject;
determining a wellbeing state of a third subject;
determining whether to alert one or more of the second subject and the third subject based on the state of one or more of the first subject, the second subject and the third subject; and
in response to determining whether to alert one or more of the second subject and the third subject, actuating one or more of a first alarm for alerting the second subject and a second alarm for alerting the third subject.

12. A method as claimed in claim 11, further comprising:

receiving user preferences;
reading historic data from a memory unit;
determining the temperature of the first subject; and
determining whether to alert one or more of the second subject and the third subject further based on one or more of the user preferences, the historic data and the temperature of the first subject.

13. A method as claimed in any of claims 11 or 12, further comprising:

determining the sleep stage of any one of the first subject, the second subject and the third subject; and
determining whether to alert one or more of the second subject and the third subject further based on the sleep stage of one or more of the first subject, second subject and the third subject.

14. A method as claimed in any of claims 10 to 13, further comprising selecting an alert to be actuated by an alarm based on the state of the first subject.

15. A computer program comprising code means for implementing the method of any one of claims 10 to 14 when said program is run on a processing system.

FIG.1

FIG.2

FIG.3

402b

402a

424

426

422

| Child OK | Cover Child | Child OK | NEEDS SPACE | Child OK | Child in unrest | Child OK |
|----------|-------------|----------|-------------|----------|-----------------|----------|

422

422

428

422

| Child present in bed | Quiet and covered | Uncovered, room temp ok | Quiet and covered | Not sufficient space | Quiet and covered | Starts to flutter | Quiet and covered |
|----------------------|-------------------|-------------------------|-------------------|----------------------|-------------------|-------------------|-------------------|

404    406    408    406    410    406    412    406

FIG.4

502 — | Child is present |

504 — | State of Child |

506 — | Alert second subject? |

508 — | Actuate alarm |

FIG.5

Europäisches
Patentamt

European
Patent Office

Office européen
des brevets

# EUROPEAN SEARCH REPORT

**Application Number**

EP 20 15 2864

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/078735 A1 (DALGLEISH MARTIN [US] ET AL) 22 March 2018 (2018-03-22) * paragraphs [0029] - [0036], [0051], [0055], [0059] - [0064]; claims; figures * ----- | 1-15 | INV. A61B5/00 ADD. A61B5/0205 A61B5/11 G08B21/02 |
| X | US 2017/206766 A1 (CHILD MICHAEL D [US] ET AL) 20 July 2017 (2017-07-20) * paragraphs [0045] - [0046], [0050] - [0056], [0068] - [0078], [0083] - [0090]; claims; figures * ----- | 1-15 | |
| X | US 2017/356992 A1 (SCHOLTEN ULRICH [FR] ET AL) 14 December 2017 (2017-12-14) * paragraphs [0016], [0018], [0035], [0048], [0061], [0064], [0068] - [0082], [0094]; claims; figures * ----- | 1-15 | |
| X | US 2018/028111 A1 (WARIS MIKKO [FI] ET AL) 1 February 2018 (2018-02-01) * paragraphs [0066], [0068], [0070] - [0078], [0083], [0088] - [0095], [0102] - [0105], [0116] - [0119], [0130]; claims; figures * ----- | 1-15 | TECHNICAL FIELDS SEARCHED (IPC) A61B G08B |

The present search report has been drawn up for all claims

| Place of search | Date of completion of the search | Examiner |
|---|---|---|
| The Hague | 16 July 2020 | Crisan, Carmen-Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 15 2864

This annex lists the patent family members relating to the patent documents cited in the above-mentioned European search report.
The members are as contained in the European Patent Office EDP file on
The European Patent Office is in no way liable for these particulars which are merely given for the purpose of information.

16-07-2020

| Patent document cited in search report | | | Publication date | Patent family member(s) | | Publication date |
|---|---|---|---|---|---|---|
| US 2018078735 | A1 | | 22-03-2018 | US 2018078735 A1 | | 22-03-2018 |
| | | | | US 2020038623 A1 | | 06-02-2020 |
| US 2017206766 | A1 | | 20-07-2017 | US 10410496 B1 | | 10-09-2019 |
| | | | | US 2017206766 A1 | | 20-07-2017 |
| US 2017356992 | A1 | | 14-12-2017 | EP 2923642 A1 | | 30-09-2015 |
| | | | | US 2015276925 A1 | | 01-10-2015 |
| | | | | US 2017356992 A1 | | 14-12-2017 |
| US 2018028111 | A1 | | 01-02-2018 | AU 2016214265 A1 | | 17-08-2017 |
| | | | | AU 2020200156 A1 | | 30-01-2020 |
| | | | | CN 107205660 A | | 26-09-2017 |
| | | | | EP 3229671 A1 | | 18-10-2017 |
| | | | | US 2018028111 A1 | | 01-02-2018 |
| | | | | WO 2016124817 A1 | | 11-08-2016 |

EPO FORM P0459

For more details about this annex : see Official Journal of the European Patent Office, No. 12/82